# EUROPÄISCHE PATENTANMELDUNG

(11) **EP 1 568 348 A1**
(43) Veröffentlichungstag der Anmeldung: **31.08.2005**
(21) Anmeldenummer: 04028292.3
(22) Anmeldetag: 01.12.2004
(51) Int. Cl.: A61K 7/06, A61K 7/08

(54) **Verfahren zur dauerhaften Ausrüstung keratinischer Fasern mit Pflegewirkstoffen durch Carboxylesterhydrolasen**

(30) Priorität: 03.12.2003 DE 10356494
(71) Anmelder: Henkel Kommanditgesellschaft auf Aktien, 40589 Düsseldorf (DE)
(72) Erfinder: Schulze zur Wiesche, Erik, 20251 Hamburg (DE); Kleen, Astrid, 22763 Hamburg (DE); Hollenberg, Detlef, 40699 Erkrath (DE); Sättler, Andrea, 40225 Düsseldorf (DE); Otto, Ralf, 88422 Oggelshausen (DE); Siegert, Petra, 42781 Haan (DE); Bossmann, Britta, 40699 Erkrath (DE)

(57) **Zusammenfassung**

Die vorliegende Erfindung betrifft ein Verfahren zur dauerhaften Ausrüstung keratinischer Fasern mit Pflegewirkstoffen durch Carboxylesterhydrolasen.

## Beschreibung

Die vorliegende Erfindung betrifft ein Verfahren zur dauerhaften Ausrüstung keratinischer Fasern mit Pflegewirkstoffen durch Carboxylesterhydrolasen.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle. Dieses Verhalten führt dazu, daß Haare in vielfältiger Weise strapazierenden Einflüssen ausgesetzt sind, die sich negativ auf die Oberflächenstruktur auswirken.

Es hat daher nicht an Anstrengungen gefehlt, der Schädigung der Haarstruktur durch Einsatz von Pflegewirkstoffen entgegenzuwirken bzw. vorzubeugen. Es wurden vielfältige Pflegekomponenten entwickelt, die gezielt als Nachbehandlungsmittel für geschädigtes Haar zum Einsatz kommen. Ferner wurden die Haarbehandlungsmittel an sich, wie beispielsweise die Fixiermittel im Rahmen einer Dauerwellbehandlung oder die Färbemittel, mit zusätzlichen Pflegestoffen versetzt. So wurde beispielsweise in der DE-A1-196 17 569 vorgeschlagen, spezielle Aminosäuren als Pflegestoffe zu verwenden.

Aus der DE-A-199 45 487 ist ein Verfahren zur Restrukturierung keratinischer Fasern bekannt, bei dem auf Fasern mindestens ein Enzym vom Typ der Carboxylesterhydrolasen und mindestens ein Wirkstoff, der eine Substrataktivität für das Enzym aufweist, aufgebracht werden.

Die EP-A-0 713 696 beschreibt ein Lipase enthaltendes Produkt zur topischen Anwendung, das befähigt sein soll, auf der Haut eine Hydroxysäure freizusetzen und seine Verwendung zur sanften Behandlung der Haut, einschließlich der Kopfhaut.

Aus der EP-A-0 530 865 ist eine Lipase enthaltende Zusammensetzung zur Reinigung von Haut, Kopfhaut und Haaren bekannt.

Überraschenderweise wurde nun gefunden, daß Carboxylesterhydrolasen und insbesondere Lipasen eingesetzt werden können, um die Oberfläche von Haaren mit Pflegewirkstoffen dauerhaft auszurüsten.

Gegenstand der vorliegenden Erfindung ist somit ein Verfahren zur dauerhaften Ausrüstung keratinischer Fasern mit Pflegewirkstoffen durch Carboxylesterhydrolasen, das dadurch gekennzeichnet ist, daß man auf die Fasern
a) mindestens ein Enzym vom Typ der Carboxylesterhydrolasen und
b) mindestens einen Pflegewirkstoff, der eine Substrataktivität für das Enzym aufweist, aufbringt.

Unter Pflegewirkstoffen im Sinne der Erfindung sind solche Wirkstoffe zu verstehen, die den durch verschiedenartigste Einflüsse entstehenden Schädigungen keratinischer Fasern entgegenwirken bzw. diesen Schädigungen vorbeugen. Hierbei spielt beispielsweise die Wiederherstellung der natürlichen Festigkeit eine wesentliche Rolle. Beispiele für schädigende Einflüsse sind Dauerwellbehandlungen, oxidative Färbungen oder Aufhellungen der Haare sowie häufiges Waschen, Fönen und Kämmen. Weiterhin können Schädigungen durch Umwelteinflüsse, wie beispielsweise UV-Licht, auftreten.

Erfindungsgemäß ausgerüstete Fasern zeichnen sich beispielsweise durch einen verbesserten Glanz, durch einen verbesserten Griff und/oder durch eine leichtere Kämmbarkeit aus. Ferner läßt sich eine erfolgreiche Ausrüstung physikalisch als Schmelzpunktserhöhung im Vergleich zur geschädigten Faser nachweisen.

Das erfindungsgemäße Verfahren führt außerdem zur Wiederherstellung der natürlichen Hydrophobie der Haaroberfläche. Mit zunehmender Haarschädigung wird die Haaroberfläche zunehmend hydrophiler. Durch die hier beschriebene Behandlung erhält das Haar seine wasserabweisende Oberflächenausrüstung zurück (Imprägnierung).

Neben der beschriebenen Wirkung auf geschädigte Fasern ist ein zweiter Aspekt der vorliegenden Erfindung auch die Erzielung einer haarfestigenden Wirkung bei ungeschädigtem Haar. Das erfindungsgemäße Verfahren kann daher auch zur Festigung der Haare für spezielle Stylingeffekte angewendet werden.

Unter keratinischen Fasern werden erfindungsgemäß Pelze, Wolle, Federn und insbesondere menschliche Haare verstanden.

In dem erfindungsgemäßen Verfahren sind grundsätzlich alle Carboxylesterhydrolasen einsetzbar, insbesondere die unter E.C.3.1.1.- Carboxylic ester hydrolases klassifizierten Enzyme, die beispielsweise im Internet unter der URL http://www.biochem.ucl.ac.uklbsmlenzymes/ec3/ec01/ec01/index.html aufgelistet sind.

Bevorzugte Carboxylesterhydrolasen sind erfindungsgemäß die Lipasen (E.C.3.1.1.3 Triacylglycerol lipase), die z. B. unter der URL
http://www.biochem.ucl.ac.uk/bsm/enzymes/ec3/ec01/ec01/ec0003/index.html zu finden sind.

Industriell erhältliche oder eingesetzte Lipasen stammen gegenwärtig beispielsweise aus den Pilzen Humicola lanuginosa, Candida cylindracea, Rhizopus arrhizus, Aspergillus niger und Geotrichum candidum, aus Bakterien der Gattung Pseudomonas spec. und Chromobacterium viscosum sowie aus Schweinepankreas und Weizenkeimgewebe.

Die wichtigsten biotechnologisch eingesetzten Lipasebildner sind Mikroorganismen. Sowohl bei den Pilzen wie bei den Bakterien wurden einige Arten intensiver untersucht:

### Lipasebildner unter den Pilzen:

Es gibt mehr als 300 Pilzspecies, von denen bekannt ist, daß sie Lipasen bilden. Von besonderem Interesse sind erfindungsgemäß Pilze aus der Familie der Mucoraceae, vor allem der Gattungen (Rhizo)Mucor und Rhizopus. Sie gehören zu den Zygomyceten (Jochpilzen), landbewohnenden niederen Pilzen mit unseptierten Hyphen, die bei Sauerstoffmange) ein hefeartiges Wachstum aufweisen. Ihre Zellwand enthält Chitin. In diese Gruppe gehören bekannte Arten wie der gemeine Brotschimmel Rhizopus nigricans und der gemeine Köpfchenschimmel Mucor mucedo. Das vegetative Mycel ist haploid.
Lipasegene der Gattung Mucor gibt es sowohl konstitutiv als auch induzierbar. Das Molekulargewicht der Mucor-Lipasen liegt um 30 kDa, sie zeigen maximale Aktivität bei Fettsäurekettenlängen zwischen C8 und C12. Das Temperaturoptimum der Mucor-Lipasen liegt generell um 38°C. Die Lipasen der Gattung Rhizopus zeigen 1,3-Regiospezifität und eignen sich dadurch für verschiedene industrielle Anwendungen. Sie haben ein Temperaturoptimum zwischen 30 und 40°C. Die Lipase von R. delemar weist beispielsweise ein Temperaturoptimum von 30°C auf. Sie zeigen maximale Aktivität bei Fettsäurekettenlängen zwischen C8 und C10. Maximale lipolytische Aktivität wurde nach fünf Tagen bei pH 6 gefunden. Das Molekulargewicht der Rhizopus-Lipasen liegt im Bereich von 40 bis 45 kDa.

### Lipase-bildende Bakterien:

Auch bei den Bakterien gibt es zahlreiche Lipasebildner. Genauer bekannt sind die Lipasen der Gattungen Pseudomonas, Bacillus und Staphylococcus. Molekulargenetische Untersuchungen wurden vor allem an Pseudomonas-Lipasen durchgeführt. An Gram-positiven Organismen wurden Staphylococcus- und Bacillus-Arten untersucht. Die Lipasen Gram-negativer und Gram-positiver Stämme unterscheiden sich deutlich voneinander.

### Lipasen Gram-negativer Bakterien:

Das durchschnittliche Molekulargewicht von Pseudomonas-Lipasen liegt bei 30 kDa. Die Homologie untereinander ist meist größer als 60%. Es gibt aber auch Ps.-Lipasen, die nur eine deutlich geringere Homologie untereinander aufweisen. Es gibt hier, wie auch bei allen anderen Lipasen, eine hochkonservierte Sequenz, ein Pentapeptid (G-X-S-X-G). Dabei handelt es sich um einen Teil des aktiven Zentrums, das in der gesamten Gruppe der Serin-Hydrolasen, zu der auch die Lipasen gerechnet werden, gefunden werden kann. Diese Serin-Hydrolasen bilden eine Gruppe von Enzymen mit ähnlicher Struktur, dem sogenannten "hydrolase-fold" sowie einem ähnlichem Reaktionsmechanismus.

### Lipasen Gram-positiver Bakterien:

Hier gibt es zwischen den einzelnen Gattungen deutliche Unterschiede.

Das aktive, reife Protein der Gattung Staphylococcus ist 44-48 kDa groß, die Sequenzhomologie der Lipasen der einzelnen Stämme im Präpro-Teil beträgt nur 25 %, die reifen Enzyme zeigen dagegen 65 %ige Homologie untereinander. Bis auf das aktive Zentrum und eine Region im N-terminalen Bereich zeigen sich keine nennenswerten Homologien mit den Pseudomonas-Lipasen. Die Lipasen der Gattung Bacillus sind mit 20 - 22 kDa deutlich kleiner als die übrigen Lipasen, die Molekulargewichte von 40-50 kDa haben. Auch in der konservierten Sequenz (G-X-S-X-G) des aktiven Zentrums gibt es Unterschiede: An Position 1 findet sich ein Alanin statt des sonst üblichen Glycins. Eine derartige katalytische Sequenz findet man auch bei der Protease Subtilisin. Besonders bevorzugt sind erfindungsgemäß Lipasen, die mittels dem Fachmann bekannter Methoden aus folgenden Organismen bzw. Geweben erhältlich sind: Candida antartica, Fraktion B; Candida rugosa; Candida rugosa, Fraktion A; Pseudomonas species; Schweinepankreas; Thermomyces lanuginosa; Mucor mihei; Alcaligenes species. Besonders bevorzugt sind außerdem aus Schweineleber gewonnene Esterasen sowie aus Schweinepankreas gewonnene Esterasen, speziell ein Gemisch aus mehreren, insbesondere aus zwei aus Schweinepankreas gewonnene Esterasen.

Erfindungsgemäß ganz besonders bevorzugt sind Lipasen aus Candida rugosa und Candida antartica, Fraktion B sowie Lipasen, die ausgewählt sind unter:
LIPB_CANAR Primary accession number P41365;
LIP1_CANRU Primary accession number P20261,
LIP2_CANRU Primary accession number P32946,
LIP5_CANRU Primary accession number P32949;
LIP_THELA Primary accession number 059952;
LIP_RHIMI Primary accession number P19515.

Diese erfindungsgemäß einsetzbaren Lipasen sind durch ihre Swissprot-Zugangsnummem (accession number) gekennzeichnet und z. B. im Internet unter der URL
http://www.ncbi.nlm.nih.gov/
abrufbar.

Unter Pflegewirkstoffen mit Substrataktiviät sind erfindungsgemäß alle Substanzen zu verstehen, die mittels der Carboxylesterhydrolasen an das Haar angelagert werden können. Dies kann beispielsweise durch Vernetzung der Pflegewirkstoffe mit Substrataktivität untereinander, das heißt durch Ausbildung einer Art Hülle um das Haar erfolgen. Dies kann aber bevorzugterweise auch durch kovalente Bindungen der Pflegewirkstoffe mit Substrataktivität an geeignete Aminosäurereste der Haare erfolgen. Hierfür sind beispielsweise Sulfonyl-, Carboxyl- und Hydroxygruppen geeignet. Besonders bevorzugt sind hier die Sulfonylgruppen, da sie auf dem Haar besonders relevant sind.

Bevorzugtermaßen wird das erfindungsgemäße Verfahren daher so durchgeführt, daß man mittels einer Lipasereaktion eine Thioesterbindung erhält (Reaktion mit einem Fettalkohol). Umgekehrt ist auch die Reaktion einer Fettsäure mit Cysteinresten möglich.

Normales (gesundes) Haar besitzt 0.3 bis 0.4 Molprozent Cysteinsäure [Zahn H., Chemie in unserer Zeit, 5, 1989]. Der Gesamtgehalt an Cystein/Cysteinsäure/Cystin beträgt 16,7+-1,5 Molprozent [Menefee E. & Friedman M., J. Protein Chem. 4, 333 (1985)]. Haarbleiche, Ammoniumthioglykolat-Dauerwelle und eine NaOH-Entkräuselungsbehandlung führen zu Cystinverlusten von 39, 10 bzw. 72% [Chao et al., J. Soc. Cosmet. Chem. 30, 401, 1979]. Es wird, wie auch durch die Photooxidation durch Licht, Cysteinsäure gebildet.

In einer ersten bevorzugten Ausführungsform der vorliegenden Erfindung werden als Pflegewirkstoffe mit Substrataktivität natürlich vorkommende Substanzen eingesetzt, insbesondere höhere Fettsäuren und Fettalkohole. Erfindungsgemäß einsetzbar sind z. B. Fettsäuren mit 8 bis 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie Fettalkohole wie beispielsweise Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie Mischungen davon.

Besonders geeignet für die Zwecke der Erfindung sind Gemische natürlich vorkommender Fettsäuren, insbesondere Fettsäuremischungen aus Pflanzenölen, bevorzugt Olivenöl. Besonders bevorzugt ist die unter der Bezeichnung Prifac AC 7987 von der Firma Uniquema GmbH & Co. KG, D-46429 Emmerich erhältliche Fettsäuremischung aus Olivenöl. Ebenfalls besonders bevorzugt ist das von der Firma Shell AG, D-22284 Hamburg unter der Bezeichnung Neodol 91 E erhältliche Fettalkoholgemisch C₉₋₁₁.

Ein weiterer Gegenstand der vorliegenden Erfindung ist ein Mittel zur dauerhaften Ausrüstung keratinischer Fasern mit Pflegewirkstoffen durch Carboxylesterhydrolasen, das dadurch gekennzeichnet ist, daß es
a) mindestens ein Enzym vom Typ der Carboxylesterhydrolasen und
b) mindestens einen Pflegewirkstoff, der eine Substrataktivität für das Enzym aufweist, enthält.

Die Pflegewirkstoffe mit Substrataktivität sind in den erfindungsgemäß verwendeten Mitteln bevorzugt in Mengen von 0,005 bis 99 Gew.-%, bezogen auf das gesamte Mittel, enthalten. Mengen von 0,01 bis 2 Gew.-% sind besonders bevorzugt. Das Massenverhältnis des Enzyms vom Typ der Carboxylesterhydrolasen zum Pflegewirkstoff mit Substrataktivität beträgt bevorzugterweise 1:40000 bis 1:1, besonders bevorzugt ist ein Massenverhältnis von 1:2000 bis 1:50.

Hinsichtlich des zeitlichen Ablaufs des Verfahrens unterliegt die Erfindung keinerlei Beschränkungen. Es ist prinzipiell möglich, zwei separate Zubereitungen, enthaltend (a) den Pflegewirkstoff mit Substrataktivität und (b) das Enzym vom Typ der Carboxylesterhydrolasen nacheinander in beliebiger Reihenfolge auf die Fasern aufzubringen. In einer bevorzugten Ausführungsform werden die zwei Komponenten (a) und (b) in dieser Reihenfolge auf die Fasern aufgebracht. Auch eine separate Anwendung der Komponenten in der Reihenfolge (b) (a) ist erfindungsgemäß. Hierbei sollte allerdings zwischen den Schritten (a) und (b), kein allzu großer zeitlicher Abstand liegen, so dass die Fasern zwischen den Schritten nicht trocknen.

Obwohl diese 2-stufigen Verfahren zu den gewünschten Effekten führen, kann es bevorzugt sein, das erfindungsgemäße Verfahren in einem 1-Schritt-Prozeß durchzuführen, da diese Prozesse einfacher anzuwenden sind. Dabei kann der Pflegewirkstoff mit Substrataktivität gemeinsam mit der Enzymzubereitung aufgebracht werden. Es ist erfindungsgemäß bevorzugt die beiden Komponenten erst unmittelbar vor der Anwendung zu mischen.

Obwohl die Enzymzubereitung prinzipiell auf dem Haar verbleiben kann, wird in einer bevorzugten Ausführungsform des erfindungsgemäßen Verfahrens die Zubereitung, die das Enzym enthält, nach einer Einwirkzeit von 1 Minute bis 2 Stunden ausgespült. Dieses Ausspülen kann mit reinem Wasser oder einem marktüblichen Shampoo erfolgen. Einwirkzeiten von 5 bis 15 Minuten haben sich in den meisten Fällen als ausreichend erwiesen.

Unabhängig von dem Ablauf des erfindungsgemäßen Verfahrens hat es sich als vorteilhaft erwiesen, die Enzymzubereitung bei einer Temperatur von 20 bis 55 °C, insbesondere von 35 bis 50°C, anzuwenden.

Die Art der Zubereitung der Enzymzubereitung unterliegt keinen prinzipiellen Einschränkungen. Erfindungsgemäß geeignet sind insbesondere wässrige, alkoholische und ölige Zubereitungen sowie deren Mischungen. Besonders bevorzugt sind wasserarme bzw. wasserfreie Zubereitungen. Es kann sich beispielsweise um Lösungen, Dispersionen, Öle und Emulsionen (Wasser in ÖI-Emulsionen, ÖI in Wasser-Emulsionen sowie multiple Emulsionen und PIT-Emulsionen) handeln. Der pH-Wert dieser Zubereitungen liegt in der Regel bei 2 bis 10, bevorzugt bei 4 bis 9 und besonders bevorzugt bei 6 bis 8.

In einer bevorzugten Ausführungsform der vorliegenden Erfindung werden die Enzymzubereitungen in Form einer verdickten Lösung formuliert. Zu diesem Zweck werden die Mittel mit Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol oder auch Poylacrylsäurepolymere angedickt. Bevorzugt werden die Enzymzubereitungen niedrigviskos formuliert.

Die Enzymzubereitungen können außer dem Enzym und ggf. dem Pflegewirkstoff mit Substrataktivität alle üblichen Bestandteile enthalten, die für die Behandlung keratinischer Fasern, insbesondere menschlicher Haare, geeignet sind. Bevorzugt sind wasserarme Zubereitungen. Unter wasserarmen Systemen werden Zubereitungen verstanden mit einem Gehalt an Wasser kleiner 5 %.

Es hat sich als vorteilhaft erwiesen, wenn die Enzymzubereitung mindestens ein Tensid enthält. Es kann sich dabei sowohl um anionische, ampholytische, zwitterionische oder nichtionogene Tenside als auch um kationische Tenside handeln. Der Fachmann kann einen eventuellen Einfluß der verschiedenen Tenside auf die Aktivität des Enzyms vom Typ der Carboxylesterhydrolasen gegebenenfalls durch einfache Vorversuche überprüfen.

In einer bevorzugten Ausführungsform der vorliegenden Erfindung wird eine Kombination aus anionischen und nichtionischen Tensiden oder eine Kombination aus anionischen und amphoteren Tensiden eingesetzt.

Es hat sich aber in Einzelfällen als vorteilhaft erwiesen, die Tenside aus amphoteren oder nichtionischen Tensiden auszuwählen.

Als anionische Tenside eignen sich in erfindungsgemäßen Mitteln alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Atkytmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quatemium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten.

Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex® , Dehyquart® und Armocare® vertrieben. Die Produkte Armocare® VGH-70, ein N,N-Bis(2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und AIkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin enthalten die erfindungsgemäß verwendeten Enzymzubereitungen bevorzugt mindestens eine Ölkomponente.

Erfindungsgemäß geeignete Ölkomponenten sind prinzipiell alle wasserunlöslichen Öle und Fettstoffe sowie deren Mischungen mit festen Paraffinen und Wachsen. Als wasserunlöslich werden erfindungsgemäß solche Stoffe definiert, deren Löslichkeit in Wasser bei 20 °C kleiner als 0,1 Gew.-% beträgt. Der Schmelzpunkt der einzelnen Öl- oder Fettkomponenten liegt bevorzugt unterhalb von etwa 40 °C. Öl- und Fettkomponenten, die bei Raumtemperatur, d. h. unterhalb von 25 °C flüssig sind, können erfindungsgemäß besonders bevorzugt sein. Bei Verwendung mehrerer Öl- und Fettkomponenten sowie ggf. festen Paraffinen und Wachsen ist es in der Regel jedoch auch ausreichend, wenn die Mischung der Öl- und Fettkomponenten sowie ggf. Paraffine und Wachse diesen Bedingungen genügt.

Eine bevorzugte Gruppe von Ölkomponenten sind pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls.

Geeignet sind aber auch andere Triglyceridöle wie die flüssigen Anteile des Rindertalgs sowie synthetische Triglyceridöle.

Eine weitere, besonders bevorzugte Gruppe erfindungsgemäß als Ölkomponente einsetzbarer Verbindungen sind flüssige Paraffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-nundecylether, Di-n-dodecylether, n-Hexyl-n-octylether, n-Octyl-n-decylether, n-Decyl-nundecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-noctylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.

Ebenfalls erfindungsgemäß einsetzbare Ölkomponenten sind Fettsäure- und Fettalkoholester. Bevorzugt sind die Monoester der Fettsäuren mit Alkoholen mit 3 bis 24 C-Atomen. Bei dieser Stoffgruppe handelt es sich um die Produkte der Veresterung von Fettsäuren mit 8 bis 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z. B. bei der Druckspaltung von natürlichen Fetten und Ölen, bei der Reduktion von Aldehyden aus der Roelen'schen Oxosynthese oder der Dimerisierung von ungesättigten Fettsäuren anfallen, mit Alkoholen wie beispielsweise Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat, Isononansäure-C16-18-alkylester (Cetiol® SN), Stearinsäure-2-ethylhexylester (Cetiol® 868), Cetyloleat, Glycerintricaprylat, Kokosfettalkohol-caprinat/-caprylat und n-Butylstearat.

Weiterhin stellen auch Dicarbonsäureester wie Di-n-butyladipat, Di-(2-ethylhexyl)-adipat, Di-(2-ethylhexyl)-succinat und Di-isotridecylacelaat sowie Diolester wie Ethylenglykoldioleat, Ethylenglykol-di-isotridecanoat, Propylenglykol-di(2-ethylhexanoat), Propylenglykoldi-isostearat, Propylenglykol-di-pelargonat, Butandiol-di-isostearat und Neopentylglykoldicaprylat erfindungsgemäß verwendbare Ölkomponenten dar, ebenso komplexe Ester wie z. B. das Diacetylglycerinmonostearat.

Die erfindungsgemäß verwendeten Enzymzubereitungen enthalten gemäß einer bevorzugten Ausführungsform einen Pflegestoff. Dieser Pflegestoff ist bevorzugt ausgewählt aus kationischen Polymeren und Silikonen.

Eine erste Gruppe von kationischen Polymeren sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt.

Unter den kationischen Polymeren sind aber die permanent kationischen Polymere bevorzugt. Als "permanent kationisch" werden erfindungsgemäß solche Polymeren bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR® 400 sind bevorzugte quaternierte Cellulose-Derivate,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil® -Quat 3270 und 3272 (Hersteller: Th. Goldschmidt; diquatemäre Polydimethylsiloxane, Quaternium-80),
- Kationische Guar-Derivate, wie insbesondere die unter den Handelsnamen Cosmedia® Guar und Jaguar® vertriebenen Produkte,
   - Polymere Dimethyldiallylammoniumsalze und deren Copolymere mit Estern und Amiden von Acrylsäure und Methacrylsäure. Die unter den Bezeichnungen Merquat® 100 (Poly(dimethyldiallylammoniumchlorid)) und Merquat® 550 (Dimethyldiallylammoniumchlorid-Acrylamid-Copolymer) im Handel erhältlichen Produkte sind Beispiele für solche kationischen Polymere,
   - Copolymere des Vinylpyrrolidons mit quaternierten Derivaten des Dialkylaminoalkylacrylats und -methacrylats, wie beispielsweise mit Diethylsulfat quatemierte Vinylpyrrolidon-Dimethylaminoethylmethacrylat-Copolymere. Solche Verbindungen sind unter den Bezeichnungen Gafquat® 734 und Gafquat® 755 im Handel erhältlich,
   - Vinylpyrrolidon-Vinylimidazoliummethochlorid-Copolymere, wie sie unter den Bezeichnungen Luviquat® FC 370, FC 550, FC 905 und HM 552 angeboten werden.
   - quaternierter Polyvinylalkohol,
   - sowie die unter den Bezeichnungen
   - Polyquaternium 2,
   - Polyquaternium 17,
   - Polyquaternium 18 und
   - Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), Polyquatemium-32, Polyquaternium-35 und Polyquaternium-37 (Handelsprodukte z. B. Salcare® SC 92 und Salcare® SC 95) bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat® ASCP 1011, Gafquat® HS 110, Luviquat® 8155 und Luviquat® MS 370 erhältlich sind.

Erfindungsgemäß bevorzugte kationische Polymere sind quaternisierte Cellulose-Derivate, polymere Dimethyldiallylammoniumsalze, Polyquaternium-27 und deren Copolymere sowie Polymere vom Typ Polyquaterniuim-2. Kationische Cellulose-Derivate, insbesondere das Handelsprodukt Polymer® JR 400, und Polymere vom Typ Polyquaternium-2, insbesondere das Handelsprodukt Mirapol® A-15, sind ganz besonders bevorzugte kationische Polymere.

Die kationischen Polymeren sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Geeignet als Pflegestoff in Kombination mit oder alternativ zu kationischen Polymeren sind auch Ampho-Polymere. Unter dem Oberbegriff Ampho-Polymere sind amphotere Polymere, d. h. Polymere, die im Molekül sowohl freie Aminogruppen als auch freie -COOH- oder SO₃H-Grupp enthalten und zur Ausbildung innerer Salze befähigt sind, zwitterionische Polymere, die im Molekül quartäre Ammoniumgruppen und -COO⁻ - oder -SO₃⁻-Gruppen enthalten, und solche Polymeren zusammengefaßt, die -COOH- oder SO₃H-Gruppen und quartäre Ammoniumgruppen enthalten. Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt. Ebenfalls bevorzugte Amphopolymere setzen sich aus ungesättigten Carbonsäuren (z. B. Acryl- und Methacryl-säure), kationisch derivatisierten ungesättigten Carbonsäuren (z. B. Acrylamidopropyl-trimethyl-ammoniumchlorid) und gegebenenfalls weiteren ionischen oder nichtionogenen Monomeren zusammen, wie beispielsweise in der deutschen Offenlegungsschrift 39 29 973 und dem dort zitierten Stand der Technik zu entnehmen sind. Terpolymere von Acrylsäure, Methylacrylat und Methacrylamidopropyltrimoniumchlorid, wie sie unter der Bezeichnung Merquat® 2001 N im Handel erhältlich sind, sind erfindungsgemäß, besonders bevorzugte Ampho-Polymere.

Erfindungsgemäß verwendbare Pflegestoffe sind weiterhin Silikonöle und Silikon-Gums, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200 und DC 1401 vertriebenen Produkte sowie die Handelsprodukte DC 344 und DC 345 von Dow Corning, Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil® -Quat 3270 und 3272 (Hersteller: Th. Goldschmidt; diquaternäre Polydimethylsiloxane, Quaternium-80) und das Handelsprodukt Fancorsil® LIM-1. Ein geeignetes anionisches Silikonöl ist das Produkt Dow Corning® 1784.

Neben dem Enzym vom Typ der Carboxylesterhydrolasen und den weiteren, oben genannten bevorzugten Komponenten können die Enzymzubereitungen prinzipiell alle weiteren, dem Fachmann für solche kosmetischen Mittel bekannten Komponenten enthalten.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- Strukturanten wie Maleinsäure, Milchsäure und Aminosäuren
- haarkonditionierende Verbindungen wie Phospholipide, beispielsweise Sojalecithin, Ei-Lecitin und Kephaline,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Panthenol und Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein weiterer Gegenstand der Erfindung ist die Verwendung von
a) mindestens einem Enzym vom Typ der Carboxylesterhydrolasen und
b) mindestens einem Pflegewirkstoff, der eine Substrataktivität für das Enzym aufweist, zur dauerhaften Ausrüstung keratinischer Fasern.

Ein weiterer Gegenstand der Erfindung ist die Verwendung von
a) mindestens einem Enzym vom Typ der Carboxylesterhydrolasen und
b) mindestens einem Pflegewirkstoff, der eine Substrataktivität für das Enzym aufweist, zur Festigung keratinischer Fasern.

Ein weiterer Gegenstand der vorliegenden Erfindung ist ein zweiteiliges Kit zur dauerhaften Ausrüstung keratinischer Fasern, das eine erste Zubereitung, enthaltend (a) einen Wirkstoff mit Substrataktivität, und eine zweite Zusammensetzung, enthaltend (b) ein Enzym vom Typ der Carboxylesterhydrolasen, enthält.

### Beispiel

Das folgende Beispiel erläutert die Erfindung, ohne sie jedoch zu beschränken:

Je 20 isolierte Haarfasern (16cm lang) wurden am wurzelnahen Ende fixiert und schrittweise vorgeschädigt. In einem ersten Schritt wurden die Fasern einer Dauerwellbehandlung mit dem Handelsprodukt Poly Lock Normal unterzogen. Dabei wurden die Strähnen zunächst 30min mit der Dauerwelllösung behandelt, gründlich gespült, mit einem Handtuch getrocknet und anschließend für 10min mit der Fixierlösung behandelt. Nach Abspülen der Fixierlösung wurden die Strähnen mit einem Föhn getrocknet. Nach der Dauerwellbehandlung wurden die Strähnen mit dem Handelsprodukt Poly Blonde Ultra für 30min bei Raumtemperatur blondiert. Anschließend wurden die Strähnen gründlich gespült und erneut getrocknet. Die Behandlungsfolge Dauerwellbehandlung/Blondierung wurde an diesen Strähnen ein zweites Mal durchgeführt. Die Strähnen wurden nach dieser Vorschädigung 14 Tage bei Raumtemperatur gelagert, bevor die eigentlichen Versuche durchgeführt wurden.

Bestimmt wurden die Hydrophobierung der vorgeschädigten Haaroberfläche durch die Enzymbehandlung mit Hilfe der dynamischen Kontaktwinkelmessung nach Wilhelmy.

Die Strähnen wurden jeweils mit einer Zubereitung gemäß Tabelle I über einen Zeitraum von 36 h in einem Schraubdeckelglas behandelt.Um sicherzustellen, dass es sich bei den gefundenen Effekten um permanente Effekte basierend auf der chemischen Anbindung der Pflegestoffe an die Haaroberfläche handelt, wurden die Haare anschließend 5x mit Ethanol und 5x mit einer 14 %igen Natriumlaurylethersulfat-Lösung gereinigt. Abschließend wurde eine dynamische Kontaktwinkelmessung nach Wilhelmy durchgeführt. Die Messwerte sind ebenfalls der Tabelle I zu entnehmen.

| Versuch | Behandlung | Gemessener Kontaktwinkel in |
|---|---|---|
| A | Keine Behandlung (Referenz) | 68,6 |
| B | Behandlung mit 10 ml Neodol 91 E¹ | 69,5 |
| C (erfindugnsgemäß) | Behandlung mit 10ml Neodol 91 E sowie 100mg Enzym² | 86,4 |

| | | |
|---|---|---|
| ¹ Gemisch aus linearen C₉-C₁₁-Fettalkoholen (INCI-Bezeichnung: C9-11 Alcohols) (Shell) | | |
| ² gewonnen aus Candida rugosa, gereinigt, der Proteingehalt der Präparationen betrug 10 bis 30 Gew.-%. | | |

Versuch C ergab eine deutliche Hydrophobierung im Vergleich zum Ausgangswert (Versuch A) sowie zu dem Versuch ohne Enzymzusatz (Versuch B).

## Patentansprüche

1. Verfahren zur dauerhaften Ausrüstung keratinischer Fasern mit Pflegewirkstoffen durch Carboxylesterhydrolasen, das **dadurch gekennzeichnet ist, daß** man auf die Fasern
a) mindestens ein Enzym vom Typ der Carboxylesterhydrolasen und
b) mindestens einen Pflegewirkstoff, der eine Substrataktivität für das Enzym aufweist, aufbringt.

2. Verfahren nach Anspruch 1, **dadurch gekennzeichnet, daß** das Enzym ausgewählt ist unter Enzymen der Klasse E.C.3.1.1, insbesondere unter Enzymen der Klasse E.C.3.1.1.3.

3. Verfahren nach einem der Ansprüche 1 oder 2, **dadurch gekennzeichnet, daß** das Enzym ausgewählt ist unter Lipasen aus Candida rugosa und Candida antartica, Fraktion B.

4. Verfahren nach einem der Ansprüche 1 bis 3, **dadurch gekennzeichnet, daß** der Pflegewirkstoff mit Substrataktivität ausgewählt ist unter Fettsäuren mit 8 bis 24 C-Atomen, insbesondere Capronsäure, Caprylsäure, 2-Ethylhexansäure, Caprinsäure, Laurinsäure, Isotridecansäure, Myristinsäure, Palmitinsäure, Palmoleinsäure, Stearinsäure, 1-sostearinsäure, Ölsäure, Elaidinsäure, Petroselinsäure, Linolsäure, Linolensäure, Elaeostearinsäure, Arachinsäure, Gadoleinsäure, Behensäure und Erucasäure.

5. Verfahren nach einem der Ansprüche 1 bis 3, **dadurch gekennzeichnet, daß** der Pflegewirkstoff mit Substrataktivität ausgewählt ist unter Fettalkoholen, insbesondere Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie Mischungen davon.

6. Verfahren nach einem der Ansprüche 1 bis 5, **dadurch gekennzeichnet, daß** das Enzym gemeinsam mit dem Pflegewirkstoff mit Substrataktivität auf die Fasern aufgebracht wird.

7. Verfahren nach einem der Ansprüche 1 bis 6, **dadurch gekennzeichnet, daß** die Einwirkzeit 3 bis 120 Minuten beträgt.

8. Verwendung von
a) mindestens einem Enzym vom Typ der Carboxylesterhydrolasen und
b) mindestens einem Pflegewirkstoff, der eine Substrataktivität für das Enzym aufweist,
zur Festigung keratinischer Fasern.

9. Verwendung von
a) mindestens einem Enzym vom Typ der Carboxylesterhydrolasen und
b) mindestens einem Pflegewirkstoff, der eine Substrataktivität für das Enzym aufweist,
zur dauerhaften Ausrüstung keratinischer Fasern mit einem Pflegestoff.

10. Mittel zur dauerhaften Ausrüstung keratinischer Fasern mit Pflegewirkstoffen durch Carboxylesterhydrolasen, das **dadurch gekennzeichnet ist, daß** es
a) mindestens ein Enzym vom Typ der Carboxylesterhydrolasen und
b) mindestens einen Pflegewirkstoff, der eine Substrataktivität für das Enzym aufweist, enthält.

11. Mittel nach Anspruch 10, **dadurch gekennzeichnet, dass** es weniger als 5 Gew.-% Wasser enthält.

12. Zweiteiliges Kit zur dauerhaften Ausrüstung keratinischer Fasern mit Pflegewirkstoffen, **dadurch gekennzeichnet, daß** es
a) eine Zusammensetzung, enthaltend einen Pflegewirkstoff mit Substrataktivität, und
b) eine Zusammensetzung, enthaltend ein Enzym vom Typ der Carboxylesterhydrolasen,
umfasst.
